(19) Europäisches Patentamt
European Patent Office
Office européen des brevets

(11) Veröffentlichungsnummer: **0 439 089 B1**

(12) # EUROPÄISCHE PATENTSCHRIFT

(45) Veröffentlichungstag der Patentschrift: **12.07.95**

(51) Int. Cl.$^6$: **C07C 25/18**, C07C 43/225, C09K 19/30, C09K 19/12, C09K 19/44, G02F 1/137

(21) Anmeldenummer: **91100675.7**

(22) Anmeldetag: **21.01.91**

(54) **Halogenierte Terphenyle und flüssigkristallines Medium.**

(30) Priorität: **25.01.90 DE 4002145**
**10.02.90 DE 4004098**
**07.03.90 DE 4007040**
**15.02.90 DE 4004649**

(43) Veröffentlichungstag der Anmeldung:
**31.07.91 Patentblatt 91/31**

(45) Bekanntmachung des Hinweises auf die Patenterteilung:
**12.07.95 Patentblatt 95/28**

(84) Benannte Vertragsstaaten:
**DE GB**

(56) Entgegenhaltungen:
**EP-A- 0 256 636**
**EP-A- 0 258 868**
**WO-A-90/09420**

(73) Patentinhaber: **MERCK PATENT GmbH**
**Postfach,**
**Frankfurter Strasse 250**
**D-64271 Darmstadt (DE)**

(72) Erfinder: **Poetsch, Eike, Dr.**
**Am Buchwald 4**
**W-6109 Mühltal 6 (DE)**
Erfinder: **Meyer, Volker, Dr.**
**Semder Pfad 23**
**W-6112 Gross-Zimmern (DE)**
Erfinder: **Bartmann, Ekkehard, Dr.**
**Dieburger Weg 12a**
**W-6106 Erzhausen (DE)**
Erfinder: **Hittich, Reinhard, Dr.**
**Am Kirchberg 11**
**W-6101 Modautal 1 (DE)**
Erfinder: **Rieger, Bernhard, Dr.**
**Hauptstrasse 31a**
**W-6115 Münster (DE)**
Erfinder: **Reiffenrath, Volker**
**Jahnstrasse 18**
**W-6101 Rossdorf (DE)**
Erfinder: **Coates, David, Dr.**
**87, Sopwith Crescent**
**Merley,**
**Wimborne,**
**Dorset BH21 3SW (GB)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 439 089 B1

Erfinder: **Greenfield, Simon, Dr.**
**2, Blackbird Close**
**Creekmoor,**
**Poole, BH17 YA (GB)**

**Beschreibung**

Die Erfindung betrifft neue halogenierte Terphenyl der Formel 1

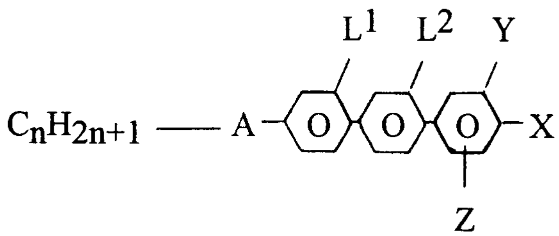

1

worin n 1 bis 7, A trans-1,4-Cycloheylen, 1,4-Phenylen oder eine Einfachbindung, X Cl oder $-CF_3$ und Y, $L^1$, $L^2$ und Z jeweils unabhängig voneinander H oder F bedeuten, wobei mindestens einer der Reste $L^1$, $L^2$ und Y F bedeutet,
der Formel 2

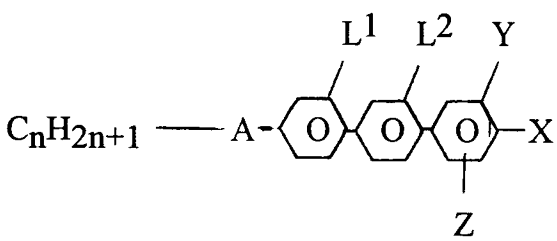

2

worin n 1 bis 7, A trans-1,4-Cyclohexylen oder 1,4-Phenylen, X F, $-OCF_3$ oder $-OCHF_2$ und $Y^1$, $L^1$, $L^2$ und Z jeweils unabhängig voneinander H oder F bedeuten, wobei mindestens einer der Reste $L^1$, $L^2$ und Y F bedeutet,
der Formel 3

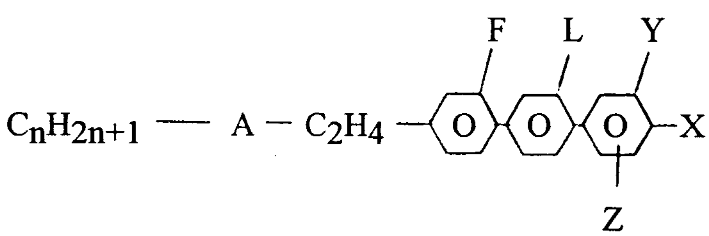

3

worin n 1 bis 7, A trans-1,4-Cyclohexylen oder 1,4-Phenylen, X F, Cl, $-CF_3$, $-OCF_3$ oder $-OCHF_2$ und Y, L und Z jeweils unabhängig voneinander H oder F bedeuten,
der Formel 4

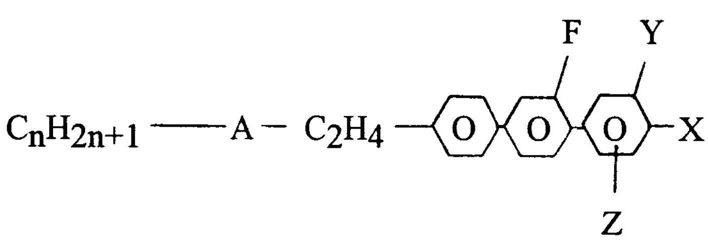

4

worin n 1 bis 7, A trans-1,4-Cyclohexylen oder 1,4-Phenylen, X F, Cl, $-CF_3$, $-OCF_3$ oder $-OCHF_2$ und Y und Z jeweils unabhängig voneinander H oder F bedeuten,
sowie der Formel 5

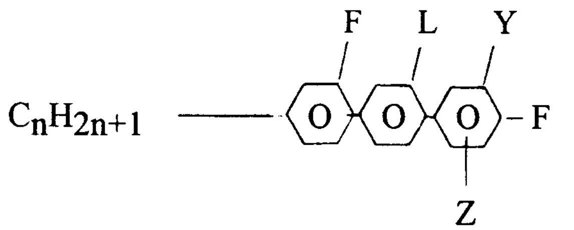

5

worin n 1 bis 7 und Y, L und Z jeweils unabhängig voneinander H oder F bedeuten, mit der Maßgabe, daß L, Y und/oder Z F bedeutet.

Aus der EP-OS 0 205 998 sind Flüssigkristalle der Formel $A^1$

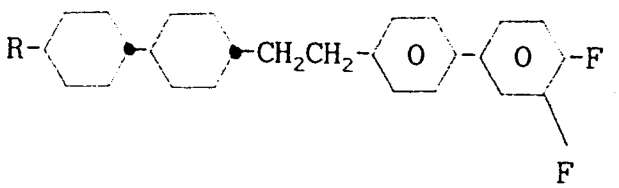

$A^1$

bekannt, worin R Alkyl mit 1 bis 10 C-Atomen bedeutet.

Die Verbindungen $A^1$ zeichnen sich nur durch eine moderate dielektrische Anisotropie aus, die in Mischungen oft zu relativ hohen Schwellenspannungen führt. Darüber hinaus ist die Mischbarkeit mit anderen Flüssigkristallverbindungen relativ schlecht.

Aus der JP-OS 60-056 932 sind Flüssigkristalle der Formel $A^2$

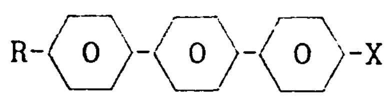

$A^2$

X = F oder Cl
bekannt.

Die Verbindungen $A^2$ zeichnen sich jedoch nur durch eine moderate dielektrische Anisotropie aus, die in Mischungen oft zu relativ hohen Schwellenspannungen führt. Darüber hinaus ist die Mischbarkeit mit und Löslichkeit in anderen Flüssigkristallverbindungen relativ schlecht und die Schmelzpunkte für eine praktische Anwendung viel zu hoch.

Diese Verbindungen der Formeln 1 - 5 können wie ähnliche, z. B. aus der DE-OS 33.17 597 bzw. 30 42 391 bekannte Verbindungen als Komponenten flüssigkristalliner Medien verwendet werden, insbesondere für Anzeigen, die auf dem Prinzip der verdrillten Zelle beruhen.

Die bisher für diesen Zweck eingesetzten Substanzen besitzen sämtliche gewisse Nachteile, beispielsweise zu hohe Schmelzpunkte, zu niedrige Klärpunkte, zu geringe Stabilität gegenüber der Einwirkung von Wärme, Licht oder elektrischen Feldern, zu niedriger elektrischer Widerstand, zu hohe Temperaturabhängigkeit der Schwellenspannung.

Insbesondere bei Anzeigen vom Supertwisttyp (STN) mit Verdrillungswinkeln von deutlich mehr als 220 ° oder bei Anzeigen mit aktiver Matrix weisen die bisher eingesetzten Materialien Nachteile auf.

Der Erfindung lag die Aufgabe zugrunde, neue flüssigkristalline Verbindungen aufzufinden, die als Komponenten flüssigkristalliner Medien geeignet sind, insbesondere für nematische Medien mit positiver dielektrischer Anisotropie, und die die Nachteile der bekannten Verbindungen nicht oder nur in geringerem Maße zeigen. Diese Aufgabe wurde durch die Bereitstellung der neuen Verbindungen der Formeln 1-5 gelöst.

Es wurde gefunden, daß die Verbindungen der Formeln 1-5 vorzüglich als Komponenten flüssigkristalliner Medien geeignet sind. Insbesondere sind mit ihrer Hilfe flüssigkristalline Medien mit weiten nematischen Bereichen, hohem Klärpunkt und optischer Anisotropie, hervorragender Nematogenität bis zu tiefen Temperaturen, hervorragender chemischer Stabilität, ausgeprägtem cl bei positiver dielektrischer Anisotropie, geringer Temperaturabhängigkeit der Schwellenspannung und/oder kleiner optischer Anisotropie erhältlich. Die neuen Verbindungen zeigen außerdem eine gute Löslichkeit für andere Komponenten derartiger Medien und hohe positive dielektrische Anisotropie bei gleichzeitig günstiger Viskosität.

Die Verbindungen der Formeln 1-5 ermöglichen sowohl STN-Anzeigen mit sehr hoher Steilheit der elektrooptischen Kennlinie als auch Anzeigen mit aktiver Matrix mit hervorragender Langzeitstabilität.

Die Verbindungen der Formeln 1-5 sind in reinem Zustand farblos und bilden flüssigkristalline Mesophasen in einem für die elektrooptische Verwendung günstig gelegenen Temperaturbereich.

Gegenstand der Erfindung sind somit die Verbindungen der Formeln 1-5 sowie die Verwendung der Verbindungen der Formeln 1-5 als Komponenten flüssigkristalliner Medien, flüssigkristalline Medien mit einem Gehalt an mindestens einer Verbindung der Formeln 1-5 und elektrooptische Anzeigen, die derartige Medien enthalten.

Vor- und nachstehend haben n, A, $L^1$, $L^2$, X, Y und Z die angegebene Bedeutung, sofern nicht ausdrücklich etwas anderes vermerkt ist.

In den Verbindungen der Formeln 1-5 sind die Alkylgruppen $C_nH_{2n+1}$ vorzugsweise geradkettig. Dementsprechend bedeutet $C_nH_{2n+1}$ Methyl, Ethyl, n-Propyl, n-Butyl, n-Pentyl, n-Hexyl oder n-Heptyl. n ist vorzugsweise 2, 3, 4 oder 5.

Verbindungen der Formeln 1-5 mit verzweigten Alkylgruppen können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Alkylreste sind Isopropyl, 2-Butyl (= 1-Methyl-propyl), Isobutyl (= 2-Methyl-propyl), 2-Methyl-butyl, Isopentyl (= 3-Methylbutyl), 2-Methylpentyl, 3-Methylpentyl oder 2-Heptyl (= 1-Methylhexyl). Der Rest

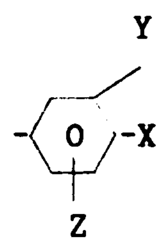

ist vorzugsweise

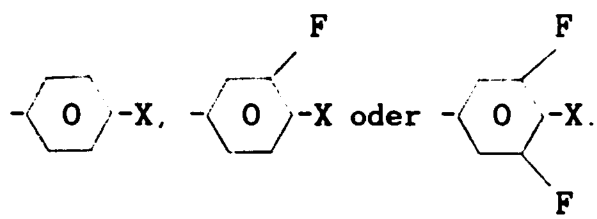

X ist vorzugsweise F, Cl, $-CF_3$ oder $-OCF_3$.

Besonders bevorzugt sind die Verbindungen der folgenden Teilformeln:

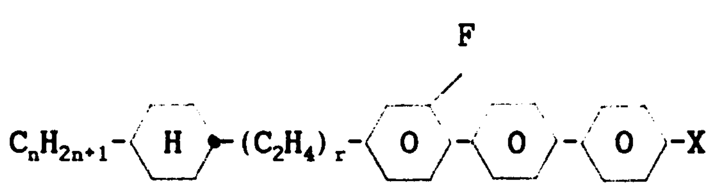

Ia

worin X Cl, $-CF_3$, $-OCF_3$ oder $-OCHF_2$ ist.

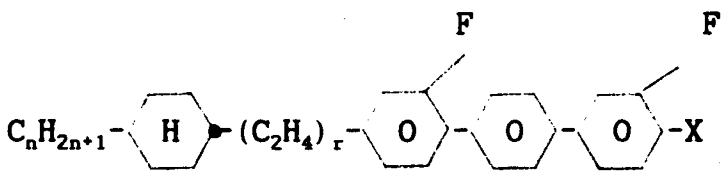

Ib

worin X F, Cl, $-CF_3$, $-OCF_3$ oder $-OCHF_2$ ist.

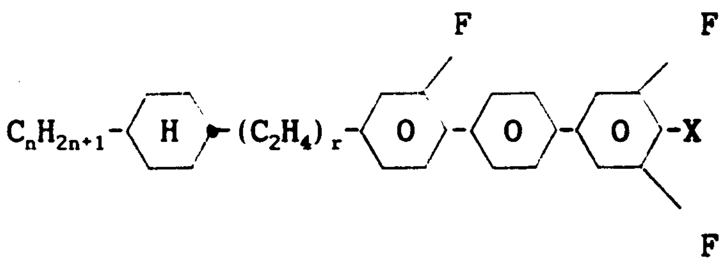

Ic

worin X F, Cl, $-CF_3$, $-OCF_3$ oder $-OCHF_2$ ist.

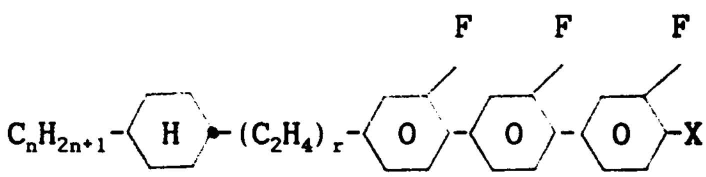

Id

worin X F, Cl, $-CF_3$, $-OCF_3$ oder $-OCHF_2$ ist.

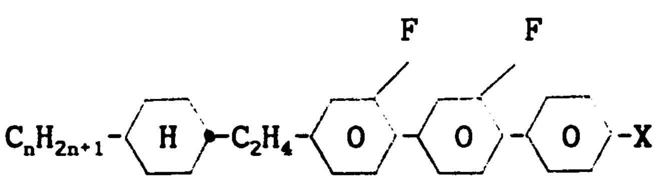

Ie

worin X F, Cl, $-CF_3$, $-OCF_3$ oder $-OCHF_2$ ist.

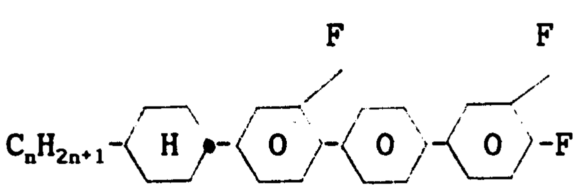

If

worin n die in Anspruch 1 angegebene Bedeutung hat.

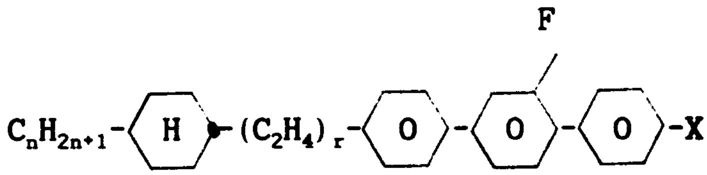

IIa

worin X F, Cl, $-CF_3$, $-OCF_3$ oder $-OCHF_2$ ist.

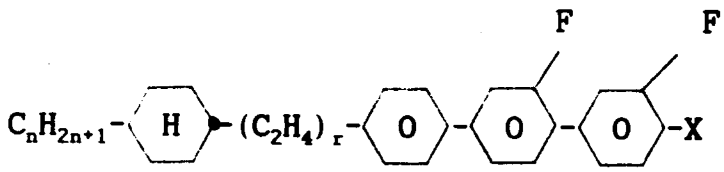

IIb

worin X F, Cl, $-CF_3$, $-OCF_3$ oder $-OCHF_2$ ist.

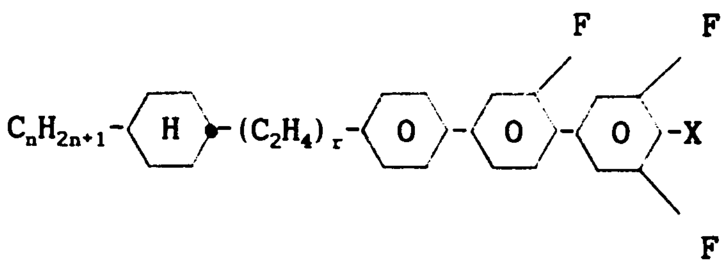

IIc

worin X F, Cl, $-CF_3$, $-OCF_3$ oder $-OCHF_2$ ist.

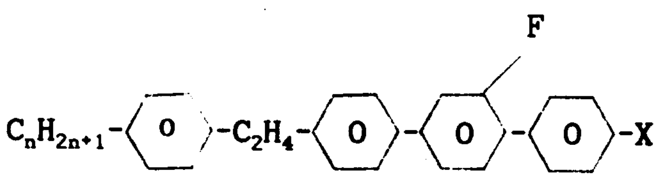

IId

worin X F, Cl, $-CF_3$, $-OCF_3$ oder $-OCHF_2$ ist.

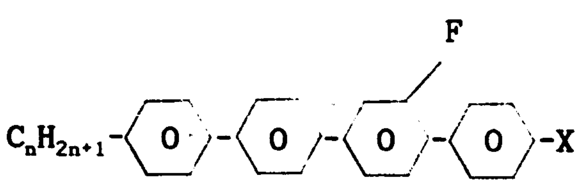

IIe

worin X F, Cl, $-CF_3$, $-OCF_3$ oder $-OCHF_2$ ist.

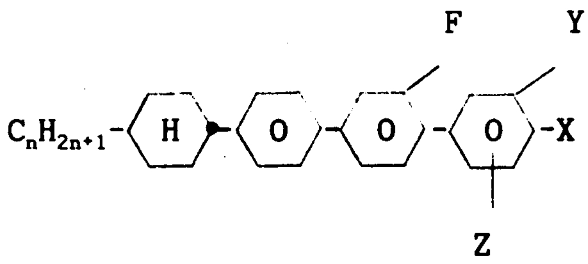

IIf

worin n, X, Y und Z die in Anspruch 1 angegebene Bedeutung haben.

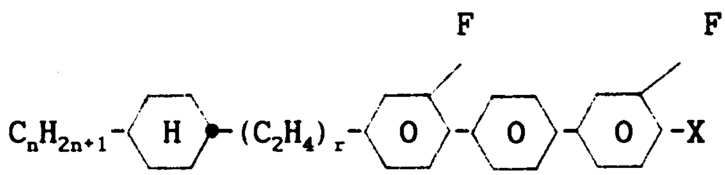

IIIa

worin X F, Cl, $-CF_3$, $-OCF_3$ oder $-OCHF_2$ ist.

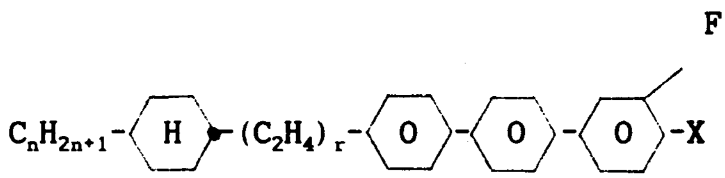

IIIb

worin X F, Cl, $-CF_3$, $-OCF_3$ oder $-OCHF_2$ ist.

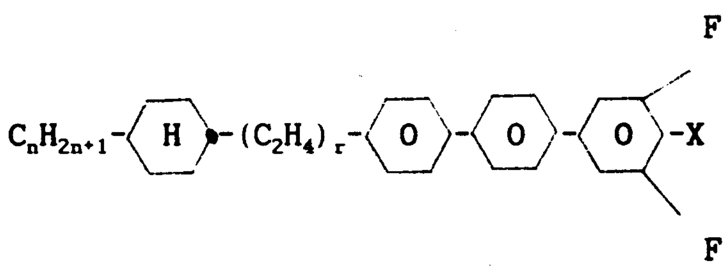

IIIc

worin X F, Cl, $-CF_3$, $-OCF_3$ oder $-OCHF_2$ ist.

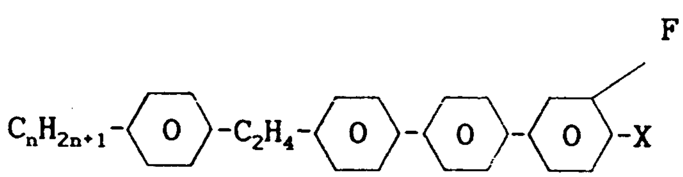

IIId

worin X F, Cl, $-CF_3$, $-OCF_3$ oder $-OCHF_2$ ist.

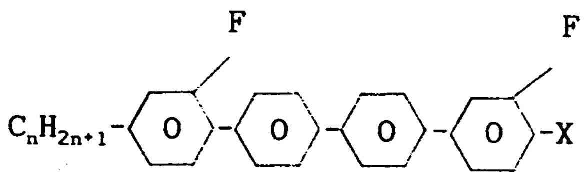

IIIe

worin X F, Cl, $-CF_3$, $-OCF_3$ oder $-OCHF_2$ ist.

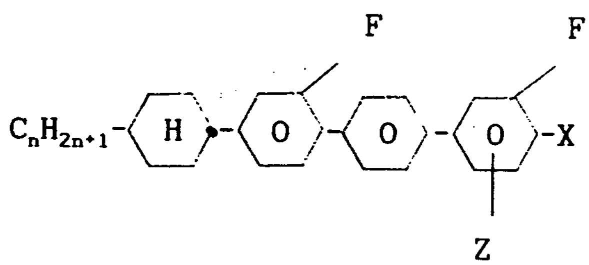

IIIf

worin n, X, Y und Z die in Anspruch 1 angegebene Bedeutung haben.

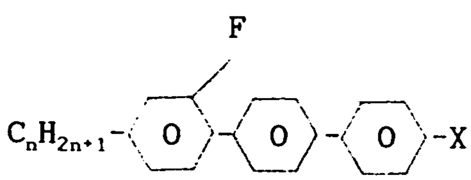

IVa

worin X Cl, oder $-CF_3$ ist.

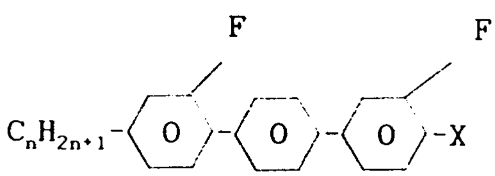

IVb

worin X F oder $-CF_3$ ist.

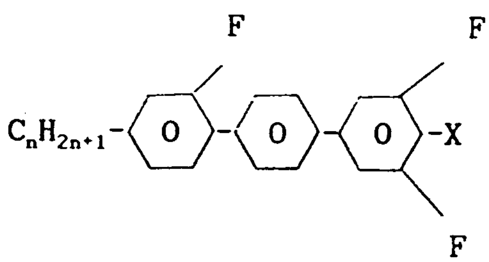

IVc

worin X F, Cl, oder $-CF_3$ ist.

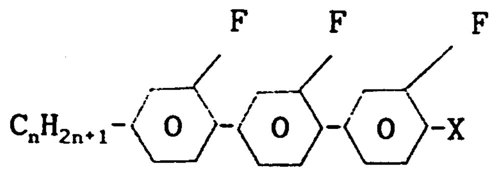

IVd

worin X F, Cl, oder $-CF_3$ ist.

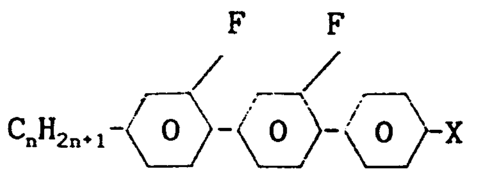

IVe

worin X F, Cl, oder $-CF_3$ ist.

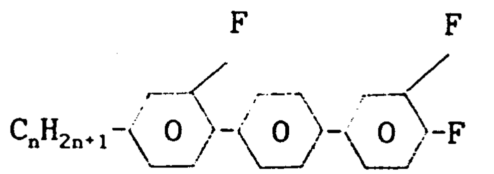

IVf

worin n die in Anspruch 1 angegebene Bedeutung hat.

Die Verbindungen der Formeln 1-5 werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formeln 1-5 umsetzt.

Die Vorstufen der Verbindungen der Formeln 1-5 werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formeln 1-5 umsetzt.

Die Verbindungen der Formeln 1-5 können durch an sich bekannte, übergangsmetallkatalysierte Kopplungsreaktionen (vgl. E. Poetsch, Kontakte 1988 (2), S. 15) z.B. nach den folgenden Syntheseschemata hergestellt werden. Die Vorstufen sind z.T. bekannt, alle Vorstufen können nach literaturbekannten Kopplungsreaktionen hergestellt werden.

Die als Ausgangsstoffe verwenden Brombiphenylderivate sind zum Teil bekannt, zum Teil können sie ohne Schwierigkeiten nach Standardverfahren der organischen Chemie aus literaturbedingten Verbindungen hergestellt werden. Beispielsweise sind die $OCF_3$- oder $OCHF_2$-Verbindungen nach bekannten Verfahren aus den entsprechenden Phenolen bzw. die $CF_3$- oder CN-Verbindungen aus den entsprechenden Benzoesäuren erhältlich. Verbindungen der Formeln 1-5

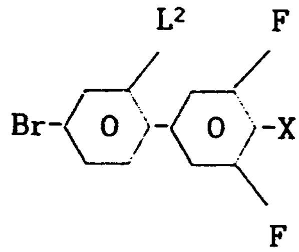

oder auch entsprechende monofluorierte Verbindungen sind beispielsweise aus den Vorstufen mit X = H durch Lithiierung bei tiefen Temperaturen und anschließende Umsetzung mit einem geeigneten Elektrophil erhältlich.

Die bevorzugten Verbindungen der Teilformel I1

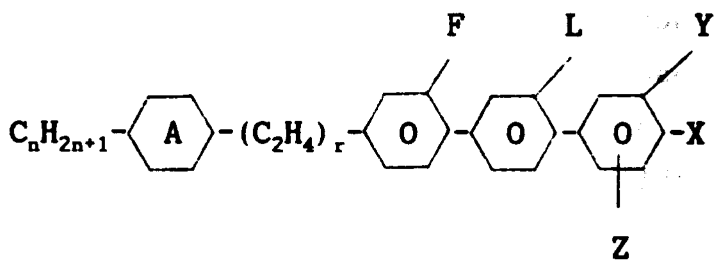

I1

worin n 1 bis 7, A trans-1,4-Cyclohexylen oder 1,4-Phenylen, r 0 oder 1, X F, Cl, $-CF_3$, $-OCF_3$ oder $-OCHF_2$ und Y, L und Z jeweils unabhängig voneinander H oder F bedeuten,
können beispielsweise wie folgt hergestellt werden:

Die Verbindungen der Formel I1, worin r = 1 bedeutet, können beispielsweise durch Umsetzung eines Aldehyds der Formel II

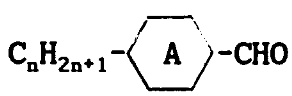

II

mit dem Phosphoniumsalz eines entsprechenden P-(subst. Phenyl)-benzylbromids nach Wittig und katalytischer Hydrierung z.B. an Pd/C der erhaltenen Ethenderivate erhalten werden.

Zur Synthese der Benzylbromide geeignete Vorstufen sind beispielsweise nach folgendem Syntheseschema erhältlich:

Schema 1a

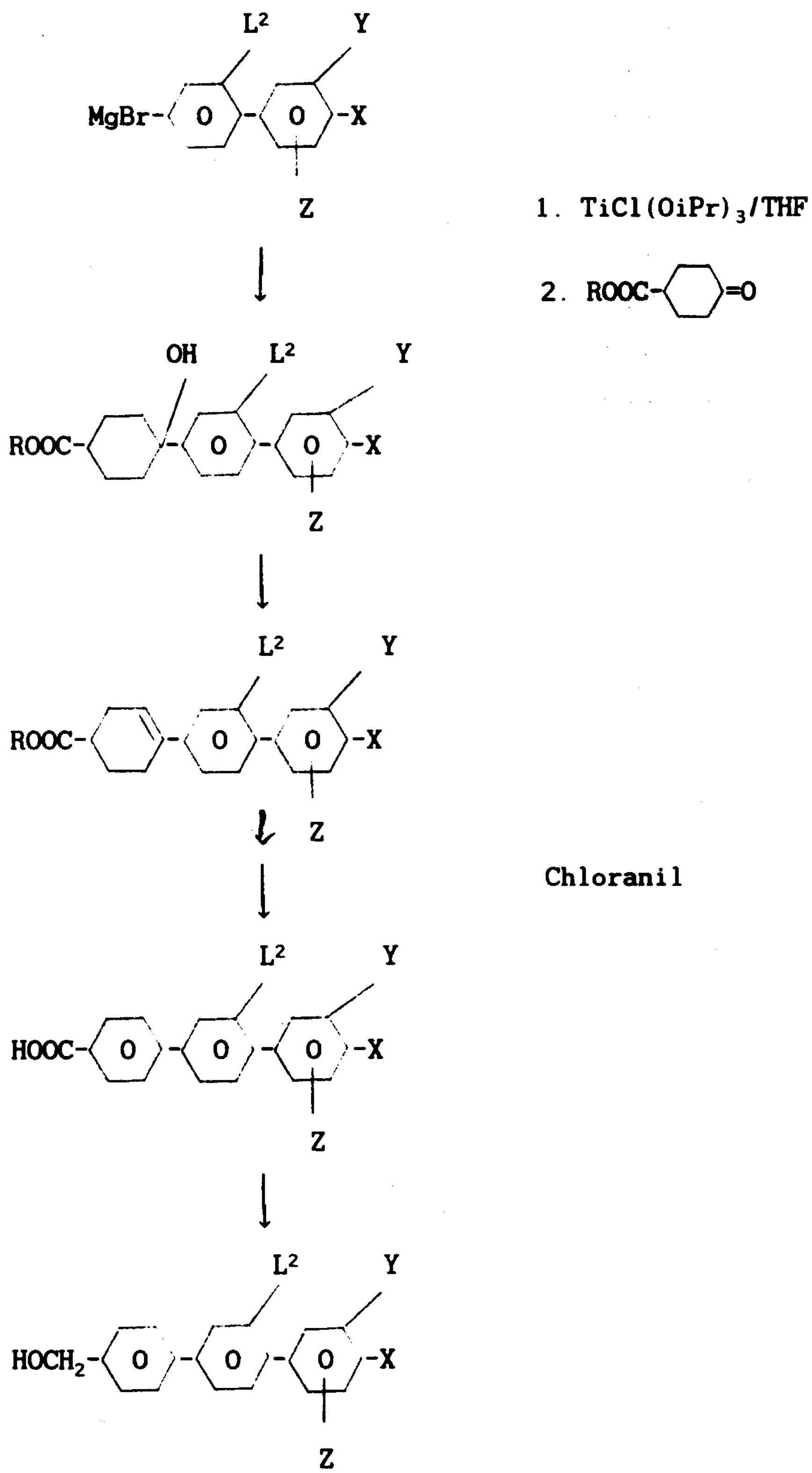


Die aus dem entsprechenden Brombiphenyl-Derivat erhaltene Grignard-Verbindung wird mit Chlortrialkylorthotitanat bzw. -zirkonat nach WO 87/05599 zu dem tertiären Cyclohexanol umgesetzt. Nach Abspaltung von Wasser, Dehydrierung und Reduktion zum Benzyalkohol erhöht man die geeignete Vorstufe.

Weitere Synthesevarianten sind dem Fachmann bekannt. Bevorzugte Varianten sind dem Schema 2a bis 4a zu entnehmen. Alle Ausgangsmaterialien sind entweder bekannt oder können in Analogie zu

bekannten Verbindungen hergestellt werden.

Schema 2a

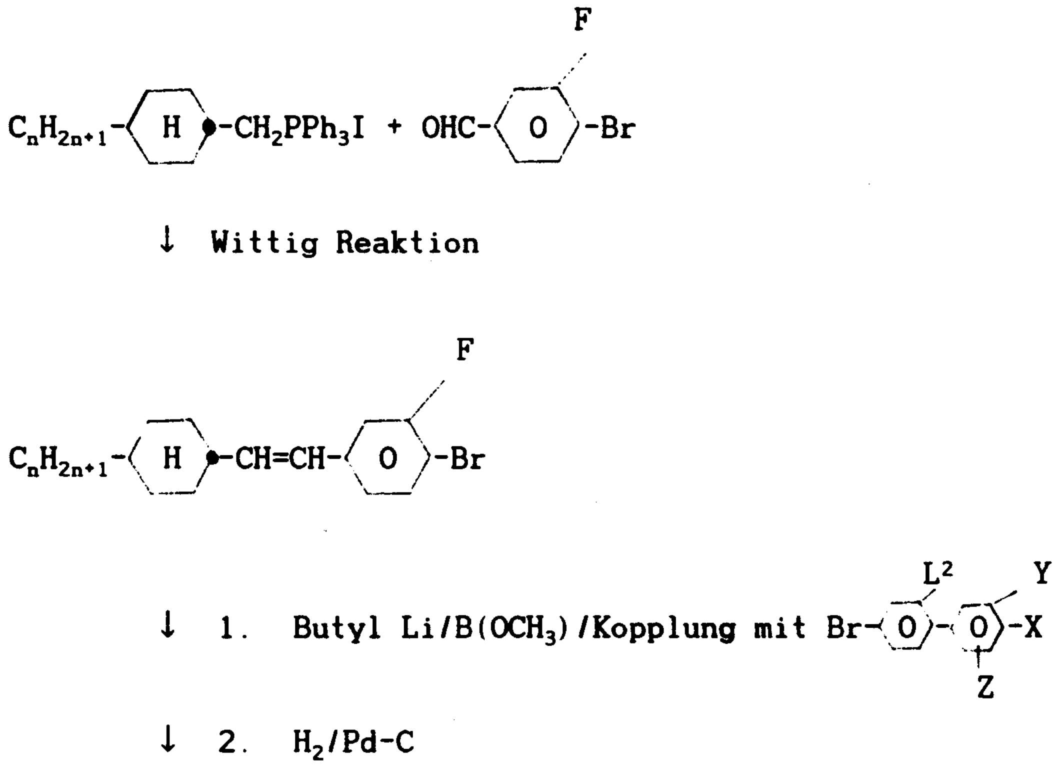

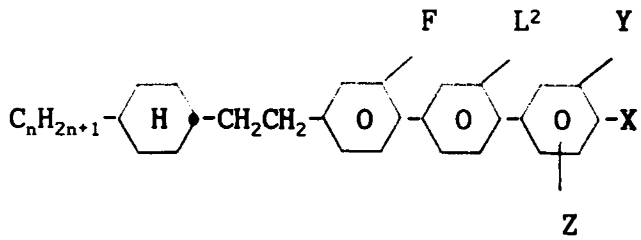

Schema 3a

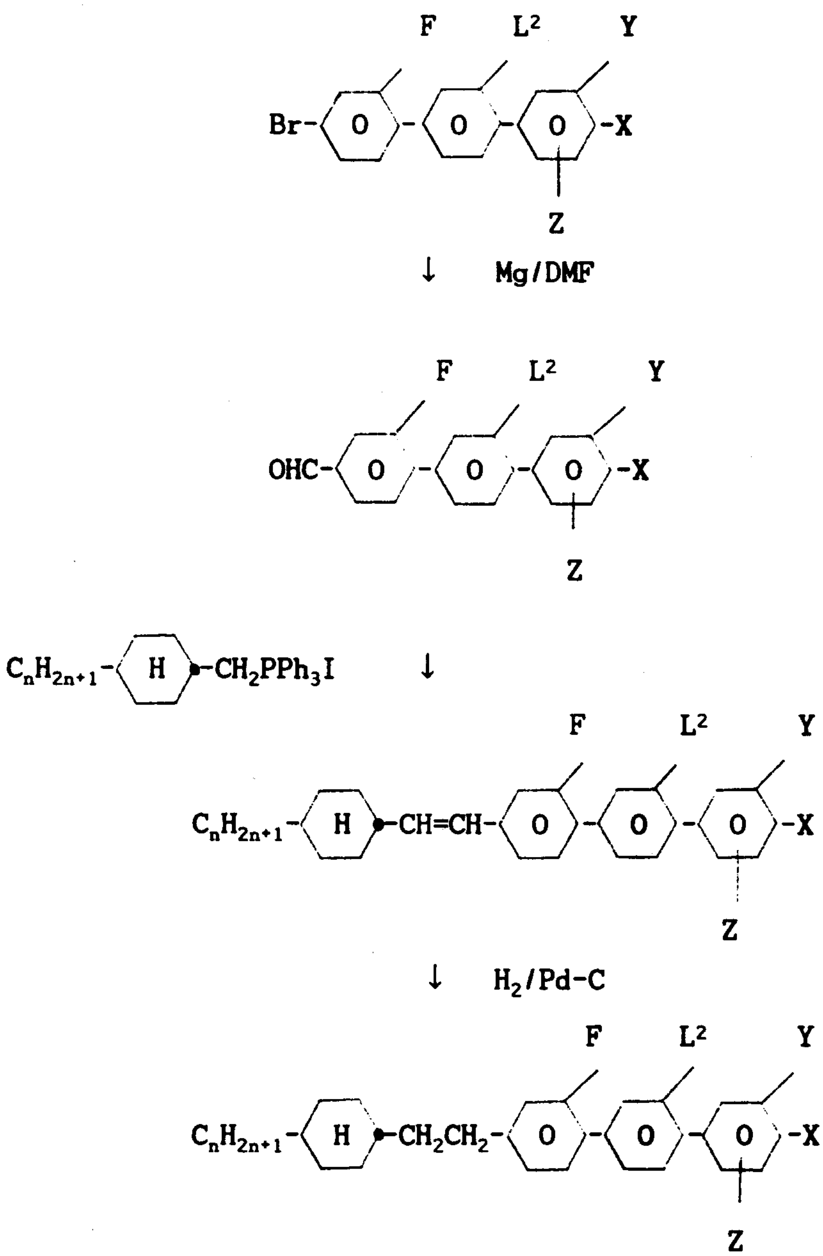

Schema 4a

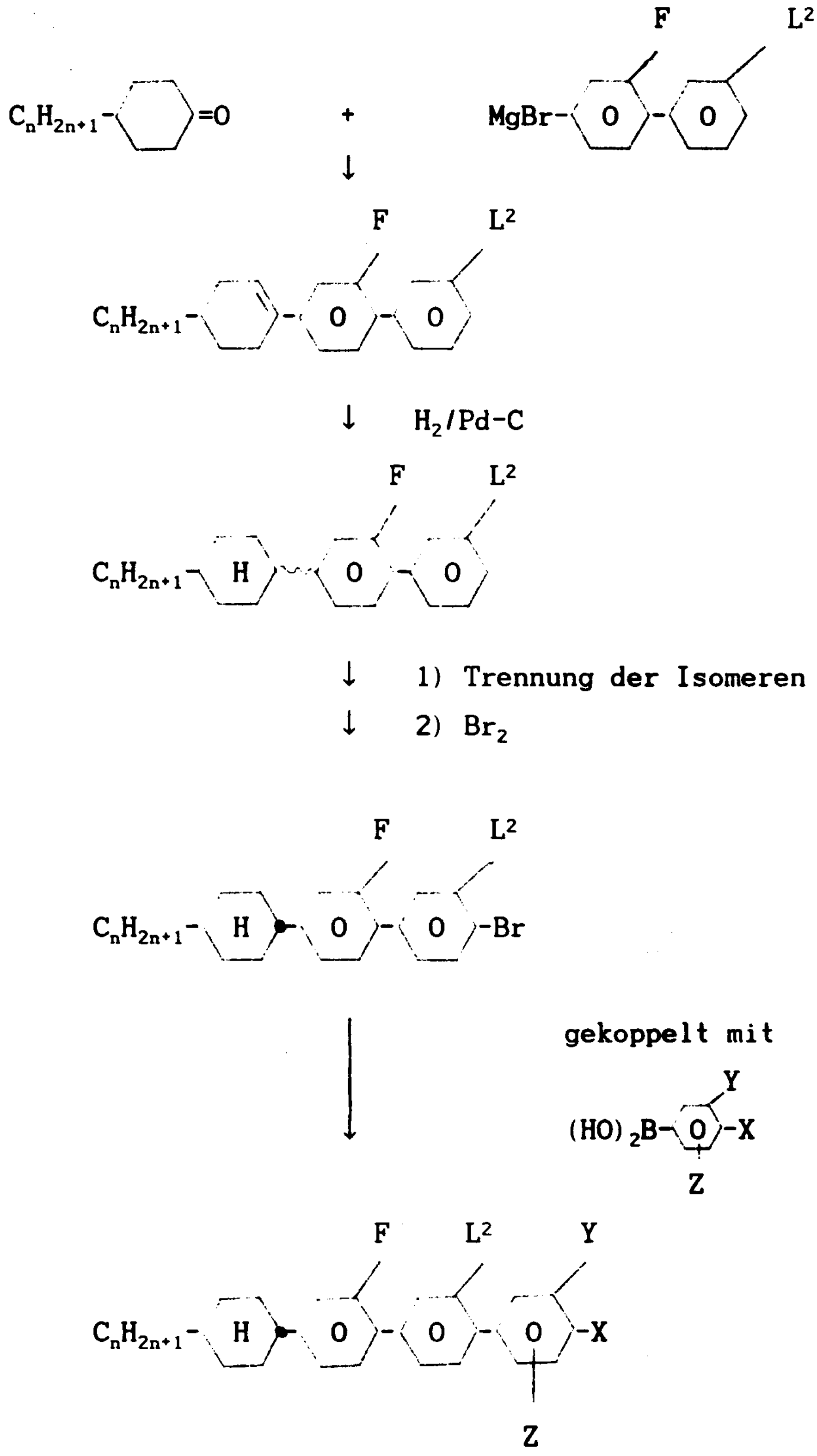

Die bevorzugten Verbindungen der Teilformel I2

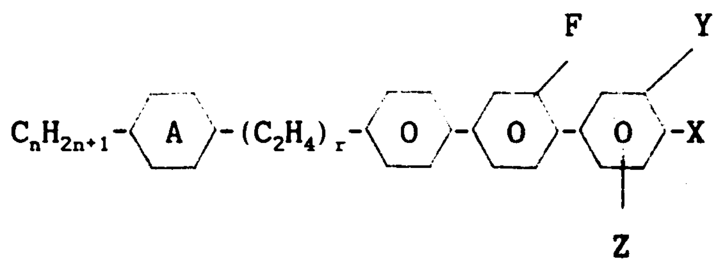

I2

worin n 1 bis 7, A trans-1,4-Cyclohexylen oder 1,4-Phenylen, r 0 oder 1, X F, Cl, $-CF_3$, $-OCF_3$ oder $-OCHF_2$ und Y, L und Z jeweils unabhängig voneinander H oder F bedeuten, sind wie folgt erhältlich:

<u>Schema 1b</u>

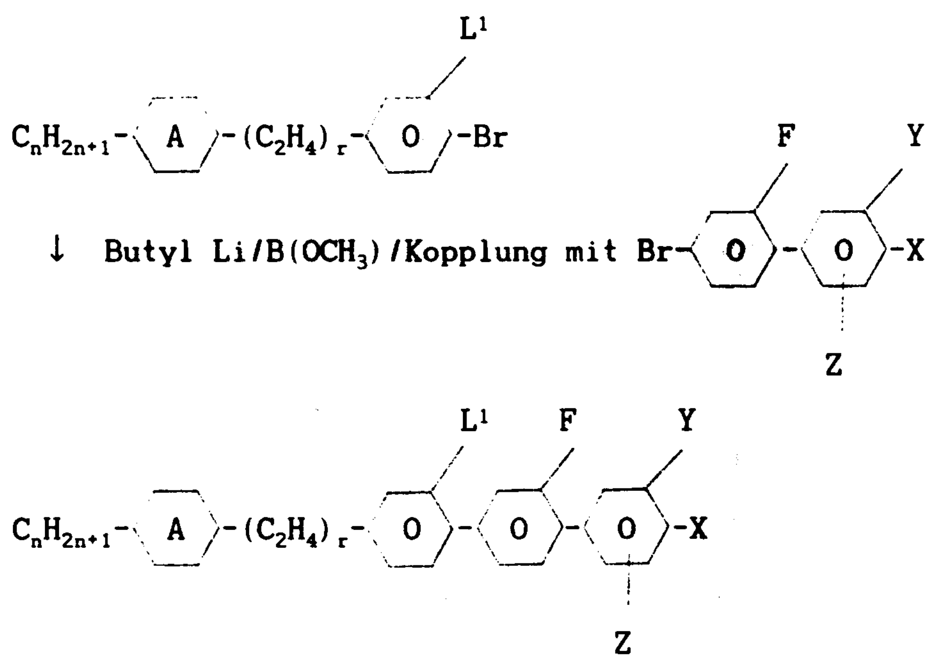

Schema 2b

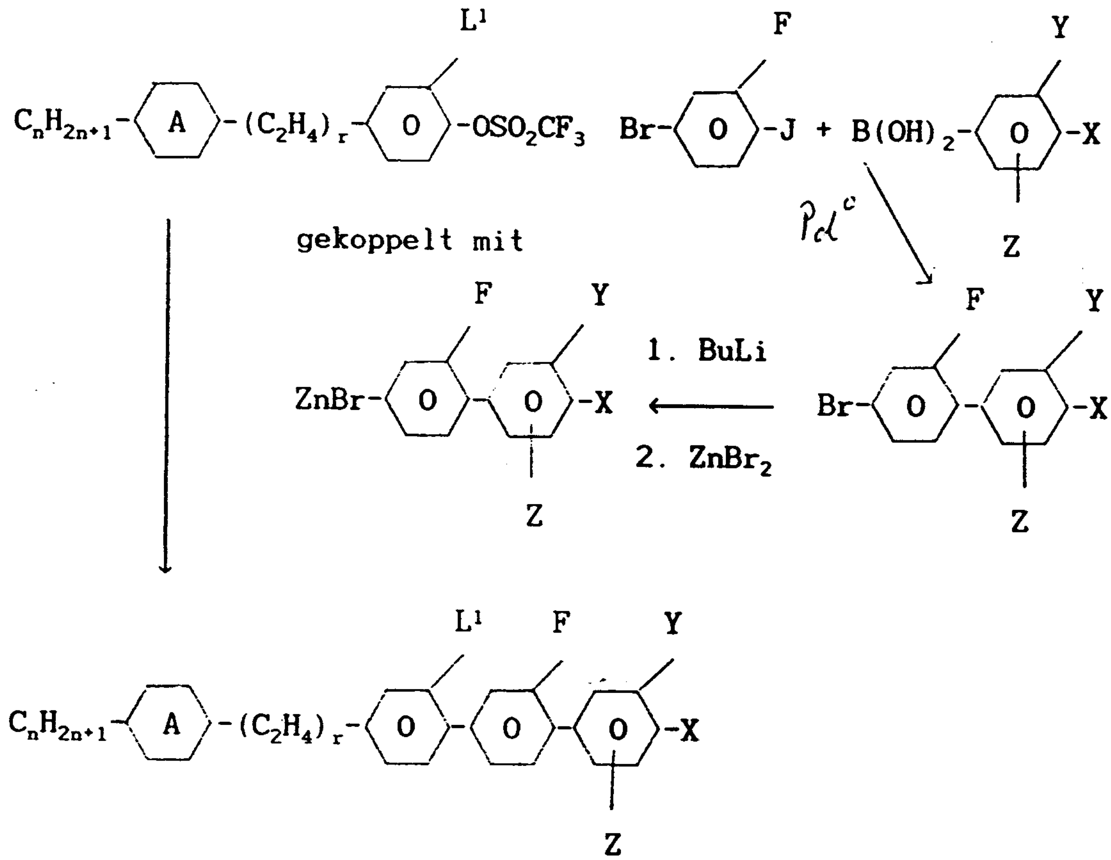


Die bevorzugten Verbindungen der Teilformel I3

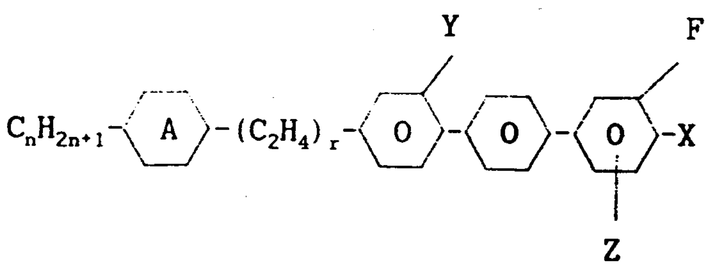

I3

worin n 1 bis 7, A trans-1,4-Cyclohexylen oder 1,4-Phenylen, r 0, X F, Cl, $-CF_3$, $-OCF_3$ oder $-OCHF_2$ und Y, L und Z jeweils unabhängig voneinander H oder F bedeuten, sind wie folgt erhältlich:

Schema 1c

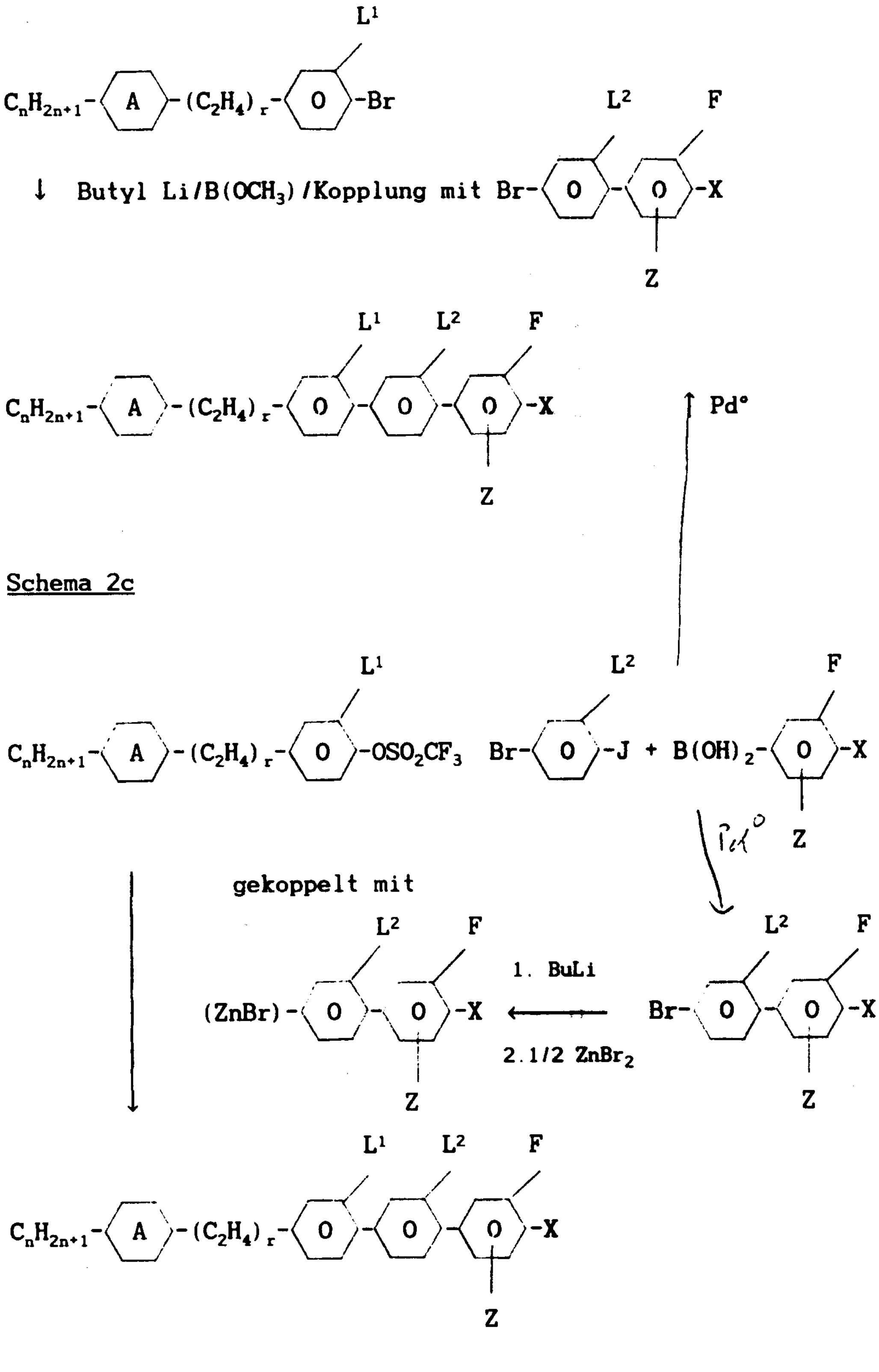


Schema 2c

Die bevorzugten Terphenyle der Formel I4

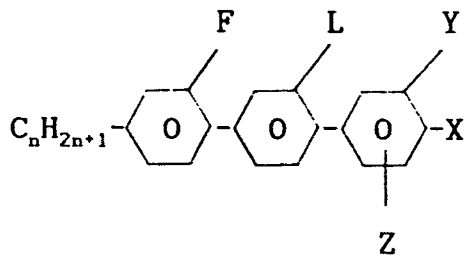

I4

worin n 1 bis 7, X Cl oder $-CF_3$ oder - im Falle L, Y und/oder Z = F - auch F, und Y, L und Z jeweils unabhängig voneinander H oder F bedeuten, sind wie folgt erhältlich:

Schema 1d

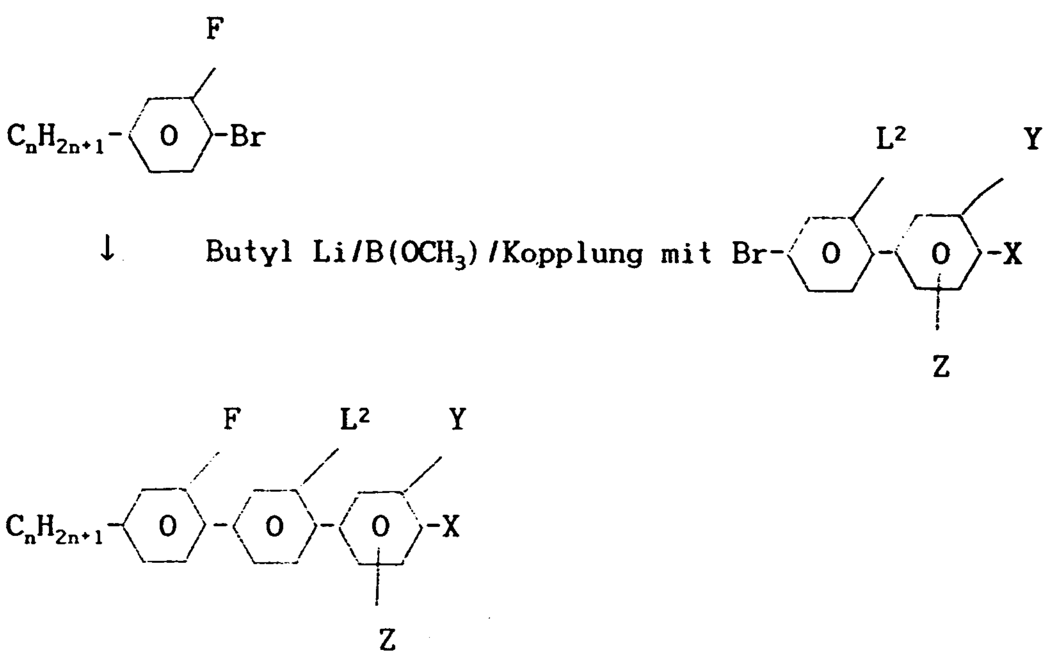

Schema 2d

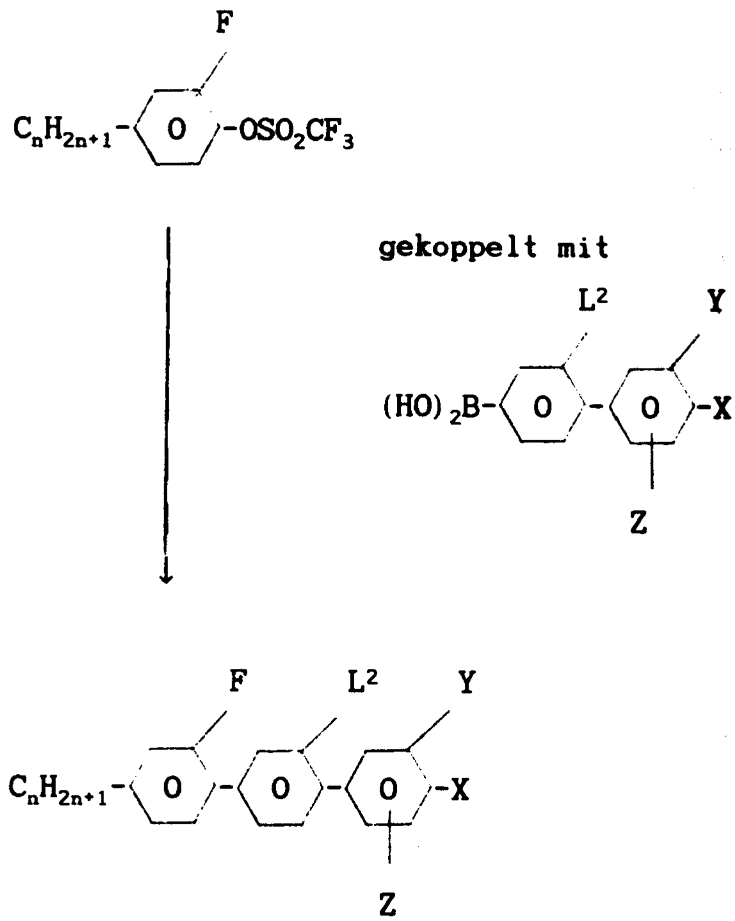


Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Biphenyle, Terphenyle, Phenyl- oder Cyclohexylbenzoate, Cyclohexan-carbonsäure phenyl- oder cyclohexyl-ester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexene, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexylphenylethane und Tolane.

Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R"    1

R'-L-COO-E-R"    2

R'-L-OOC-E-R"    3

R'-L-CH2CH2-E-R"  4

R'-L-C≡C-E-R"  5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor sub~ stituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R" bedeuten in den Verbindungen der Teilformeln 1a, 2a, 3a, 4a und 5a jeweils unabhängig voneinander Alkyl, Alkenyl, Alkoxy, Alkenyloxy oder Alkanoyloxy mit bis zu 8 Kohlenstoffatomen. Bei den meisten dieser Verbindungen sind R' und R" voneinander verschieden, wobei einer dieser Reste meist Alkyl oder Alkenyl ist. In den Verbindungen der Teilformeln 1b, 2b, 3b, 4b und 5b bedeutet R" -CN, $-CF_3$, $-OCF_3$, F, Cl oder -NCS; R' hat dabei die bei den Verbindungen der Teilformeln 1a bis 5a angegebene Bedeutung und ist vorzugsweise Alkyl oder Alkenyl. Besonders bevorzugt ist R" ausgewählt aus der Gruppe bestehend aus -F, Cl, $CF_3$ und $-OCF_3$. Aber auch andere Varianten der vorgesehenen Substituenten in den Verbin~ dungen der Formeln 1, 2, 3, 4 und 5 sind gebräuchlich. Viele solcher Substanzen oder auch Gemische davon sind im Handel erhältlich. Alle diese Substanzen sind nach literaturbekannten Methoden oder in Analogie dazu erhältlich.

Die erfindungsgemäßen Medien enthalten vorzugsweise neben Komponenten aus der Gruppe der Verbindungen 1a, 2a, 3a, 4a und 5a (Gruppe 1) auch Komponenten aus der Gruppe der Verbindungen 1b, 2b, 3b, 4b und 5b (Gruppe 2), deren Anteile vorzugsweise wie folgt sind:
Gruppe 1: 20 bis 90 %, insbesondere 30 bis 90 %,
Gruppe 2: 10 bis 80 %, insbesondere 10 bis 50 %,
wobei die Summe der Anteile der erfindungsgemäßen Verbindungen und der Verbindungen aus den Gruppen 1 und 2 bis zu 100 % ergeben.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungsgemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die folgenden Beispiele sollen die Erfindung erläutern, ohne sie zu begrenzen. mp. = Schmelzpunkt, cp. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Es bedeuten ferner:
K: Kristallin-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die

Umwandlungstemperatur in Grad Celsius an.

| | |
|---|---|
| DAST | Diethylaminoschwefeltrifluorid |
| DCC | Dicyclohexylcarbodiimid |
| DDQ | Dichlordicyanobenzochinon |
| DIBALH | Diisobutylaluminiumhydrid |
| DMSO | Dimethylsulfoxid |
| KOT | Kalium-tertiär-butanolat |
| THF | Tetrahydrofuran |
| pTSOH | p-Toluolsulfonsäure |

Beispiel 1

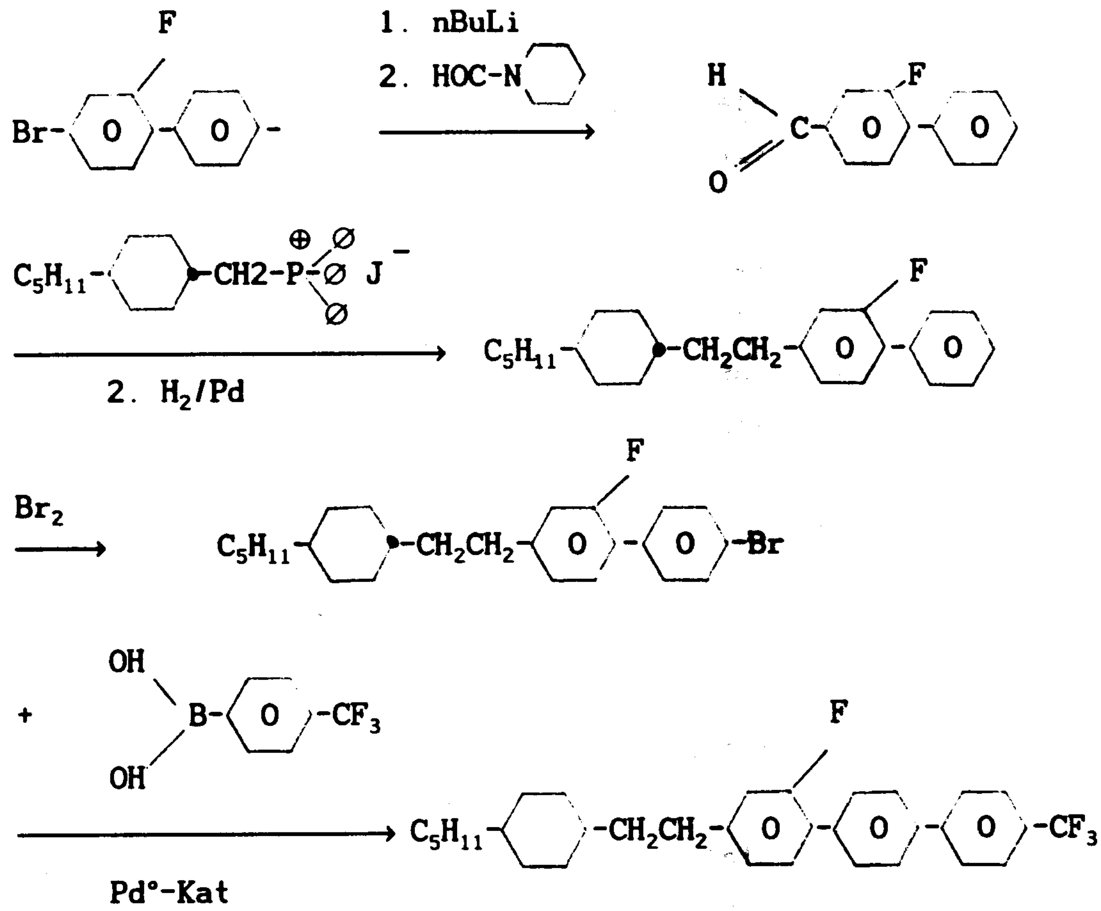


0,05 m 4'-Brom-2-fluor-4-[2-(4-pentylcyclohexyl)ethylen)-biphenyl, 0,05 m 4-Trifluormethylphenylboronsäure und 1 g Tetrakistriphenylphosphinpalladium -O Katalysator werden in 100 ml Toluol und 40 ml Ethanol gelöst und mit 50 ml 2 m-$Na_2CO_3$-Lsg. versetzt. Man kocht 4 h am Rückfluß und arbeitet danach extraktiv auf. Aus dem Rohmaterial erhält man durch Chromatographie und Kristallisation das saubere Produkt.

Beispiel 2 bis 34

Analog Beispiel 1 erhält man die folgenden Verbindungen der Teilformel I1 (A = trans-1,4-Cyclohexylen):

| | n | X | Y | Z | r | L |
|---|---|---|---|---|---|---|
| (2) | 2 | $-CF_3$ | H | H | 1 | H |
| (3) | 5 | $-CF_3$ | H | H | 1 | H |
| (4) | 4 | $-CF_3$ | H | H | 1 | H |
| (5) | 5 | F | H | H | 1 | H |
| (6) | 2 | F | H | H | 1 | H |
| (7) | 3 | F | H | H | 1 | H |
| (8) | 5 | Cl | H | H | 1 | H |
| (9) | 2 | Cl | H | H | 1 | H |
| (10) | 3 | Cl | H | H | 1 | H |
| (11) | 5 | Cl | F | H | 1 | H |
| (12) | 2 | Cl | F | H | 1 | H |
| (13) | 3 | Cl | F | H | 1 | H |
| (14) | 5 | F | H | H | 1 | F |
| (15) | 2 | F | H | H | 1 | F |
| (16) | 3 | F | H | H | 1 | F |

| | n | X | Y | Z | r | L |
|---|---|---|---|---|---|---|
| (17) | 2 | $-OCF_3$ | H | H | 1 | H |
| (18) | 3 | $-OCF_3$ | H | H | 1 | H |
| (19) | 5 | $-OCF_3$ | H | H | 1 | H |
| (20) | 2 | $-OCF_3$ | H | H | 1 | F |
| (21) | 3 | $-OCF_3$ | H | H | 1 | F |
| (22) | 5 | $-OCF_3$ | H | H | 1 | F |
| (23) | 2 | $-CF_3$ | H | H | 1 | F |
| (24) | 3 | $-CF_3$ | H | H | 1 | F |
| (25) | 5 | $-CF_3$ | H | H | 1 | F |
| (26) | 2 | F | F | H | 0 | H |
| (27) | 3 | F | F | H | 0 | H |
| (28) | 5 | F | F | H | 0 | H |
| (29) | 2 | F | H | H | 0 | F |
| (30) | 3 | F | H | H | 0 | F |
| (31) | 5 | F | H | H | 0 | F |
| (32) | 2 | Cl | H | H | 0 | F |
| (33) | 3 | Cl | H | H | 0 | F |
| (34) | 5 | Cl | H | H | 0 | F |
| (35) | 2 | F | F | H | 0 | H |
| (36) | 3 | F | F | H | 0 | H, |
| K 119 $S_A$ N 266 I | | | | | | |
| (37) | 4 | F | F | H | 0 | H |
| (38) | 5 | F | F | H | 0 | H, |
| K 97 $S_A$ 136 N 253 I | | | | | | |
| (38a) | 3 | F | F | F | 0 | H, |
| | | | | K 148 N 241 I | | |
| (38b) | 3 | $CF_3$ | H | H | 0 | H, |
| | | | K 154 $S_B$ 106 $S_A$ 240 N 280 I | | | |

Beispiel 39

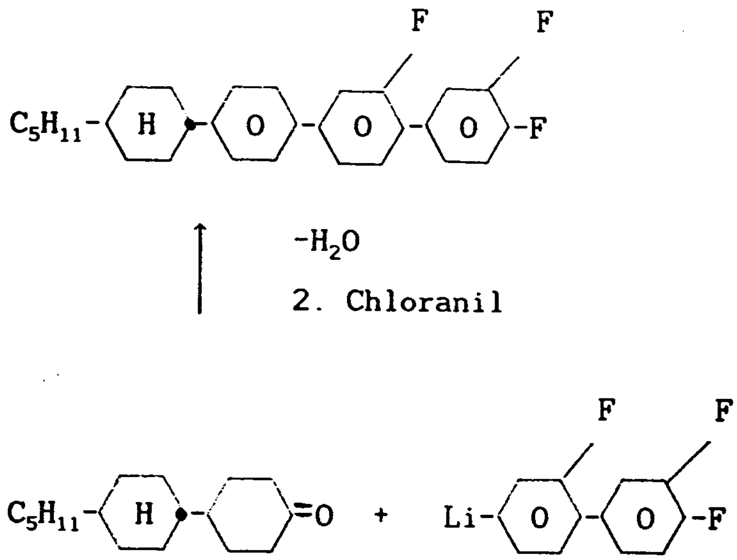


0,1 m der durch Dehydratisierung des Alkohols gewonnenen Cyclohexenverbindung (vgl. obiges Schema) werden mit 0,4 m Chloranil in 500 ml Toluol 6 h am Rückfluß gekocht. Nach extraktiver Aufarbeitung erhält man nach üblicher Aufreinigung das Zielprodukt.

Beispiel 40 bis 72

Analog Beispiel 1 erhält man die folgenden Verbindungen der Teilformel I2 (A = trans-1,4-Cyclohexylen):

| | n | X | Y | Z | r |
|---|---|---|---|---|---|
| (40) | 2 | $-CF_3$ | H | H | 1 |
| (41) | 5 | $-CF_3$ | H | H | 0 |
| (42) | 4 | $-CF_3$ | H | H | 0 |
| (43) | 5 | F | H | H | 1 |
| (44) | 2 | F | H | H | 1 |
| (45) | 3 | F | H | H | 0, K 140 $S_A$ 147 N 301 I |

| | n | X | Y | Z | r |
|---|---|---|---|---|---|
| (46) | 5 | Cl | H | H | 0 |
| (47) | 2 | Cl | H | H | 1 |
| (48) | 3 | Cl | H | H | 1 |
| (49) | 5 | Cl | F | H | 1 |
| (50) | 2 | Cl | F | H | 1 |
| (51) | 3 | Cl | F | H | 0 |
| (52) | 5 | F | H | H | 0 |
| (53) | 2 | F | H | H | 1 |
| (54) | 3 | F | H | H | 1, K 101 $S_A$ 128 N 242 I |
| (55) | 2 | $-OCF_3$ | H | H | 0 |
| (56) | 3 | $-OCF_3$ | H | H | 0, K 75 $S_B$ 136 $S_A$ 252 N > 300 I |
| (57) | 5 | $-OCF_3$ | H | H | 0 |
| (58) | 2 | $-OCF_3$ | H | H | 1 |
| (59) | 3 | $-OCF_3$ | H | H | 1, K 111 $S_B$ 117 $S_A$ 202 N 245 I |
| (60) | 5 | $-OCF_3$ | H | H | 1 |
| (61) | 2 | $-CF_3$ | H | H | 1 |
| (62) | 3 | $-CF_3$ | H | H | 1 |
| (63) | 5 | $-CF_3$ | H | H | 0 |
| (64) | 2 | F | H | H | 1 |
| (65) | 3 | F | H | H | 1 |
| (66) | 5 | F | H | H | 0 |
| (67) | 2 | F | H | H | 1 |
| (68) | 3 | F | H | H | 1 |
| (69) | 5 | F | H | H | 0 |
| (70) | 2 | Cl | H | H | 0 |
| (71) | 3 | Cl | H | H | 0 |
| (72) | 5 | Cl | H | H | 1 |

Beispiel 73

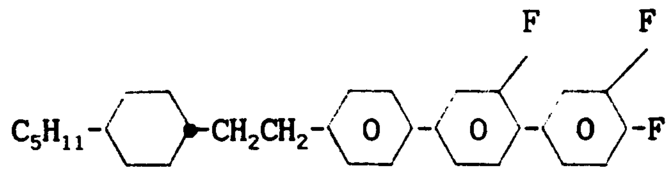

0,05 m 4'-Brom-2'-3,4-Triflourbiphenyl (erhältlich nach Schema 1), 0,05 m p-[2-(trans-4-n-Pentylcyclohexyl)-ethyl]-phenylboronsäure und 1 g Tetrakistriphenylphosphinpalladium -0 Katalysator werden in 100 ml Toluol und 40 ml Ethanol gelöst und mit 50 ml 2 m-$Na_2CO_3$-Lsg. versetzt. Man kocht 4 h am Rückfluß und arbeitet danach extraktiv auf. Aus dem Rohmaterial erhält man durch Chromatographie und Kristallisation das saubere Produkt.

Beispiel 74

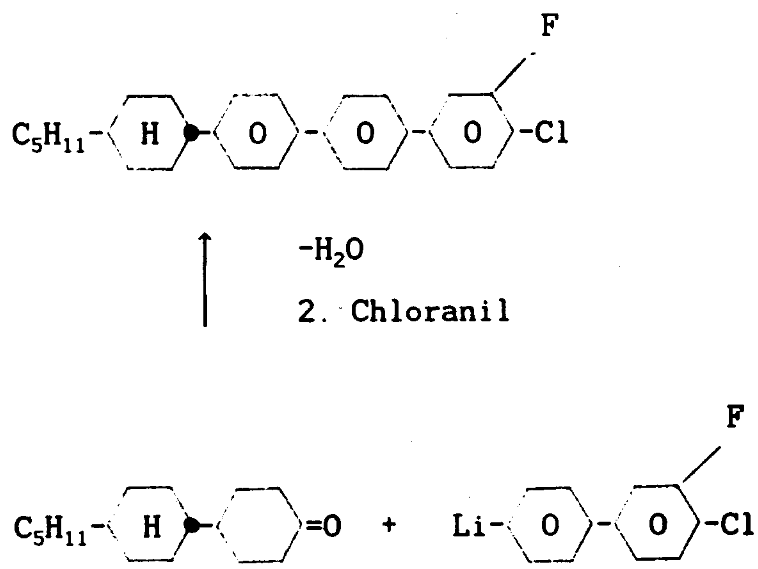


0,1 m der durch Dehydratisierung des Alkohols gewonnenen Cyclohexenverbindung (vgl. obiges Schema) werden mit 0,4 m Chloranil in 500 ml Toluol 6 h am Rückfluß gekocht. Nach extraktiver Aufarbeitung erhält man nach üblicher Aufreinigung das Zielprodukt.

Beispiel 75 bis 90

Analog Beispiel 74 erhält man die folgenden Verbindungen der Teilformel I3 (A = trans-1,4-Cyclohexylen):

| | n | X | Y | Z | r |
|---|---|---|---|---|---|
| (75) | 5 | $-CF_3$ | H | H | 0 |
| (76) | 4 | $-CF_3$ | H | H | 0 |
| (77) | 3 | F | H | H | 0 |
| (78) | 5 | Cl | H | H | 0 |
| (79) | 5 | Cl | F | H | 1 |
| (80) | 2 | Cl | F | H | 1 |
| (81) | 3 | Cl | F | H | 0 |
| (82) | 5 | F | H | H | 0 |
| (83) | 2 | $-OCF_3$ | H | H | 0 |
| (84) | 3 | $-OCF_3$ | H | H | 0 |
| (85) | 5 | $-OCF_3$ | H | H | 0 |
| (86) | 5 | $-CF_3$ | H | H | 0 |
| (87) | 5 | F | H | H | 0 |
| (88) | 5 | F | H | H | 0 |
| (89) | 2 | Cl | H | H | 0 |
| (90) | 3 | Cl | H | H | 0 |

Beispiel 91

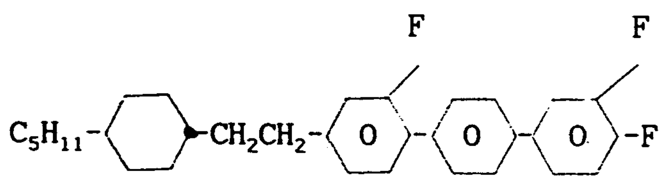

0,05 m 4'-Brom-3,4-Diflourbiphenyl (erhältlich nach Schema 1), 0,05 m p-[2-(trans-4-n-Pentylcyclohexyl)-ethyl]-2-fluor-phenylboronsäure und 1 g Tetrakistriphenylphosphin-palladium -O Katalysator werden in 100 ml Toluol und 40 ml Ethanol gelöst und mit 50 ml 2 m-$Na_2CO_3$-Lsg. versetzt. Man kocht 4 h am Rückfluß und arbeitet danach extraktiv auf. Aus dem Rohmaterial erhält man durch Chromatographie und Kristallisation das saubere Produkt.

Beispiel 92

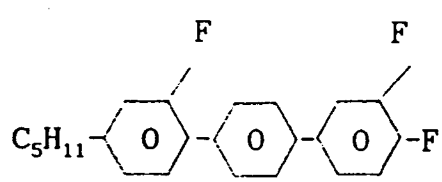

Ein Gemisch von 16,1 g 4-n-Pentyl-2-fluor-4'-brombiphenyl, 7,6 g Bis-(3,4-Difluorphenyl)-boronsäure, 65 ml 2 M wässerige $Na_2CO_3$-Lsg., 1,1 g Tetrakis(triphenylphosphin)palladium (O) und 100 ml Toluol wird 6 h refluxiert. Nach üblicher Aufarbeitung der org. Phase erhält man 4-n-Pentyl-2,3',4'-Trifluorterphenyl, K 85 N 87 I.

Beispiel 93 bis 110

Analog Beispiel 1 erhält man die folgenden Verbindungen der Teilformel I4:

| | n | X | Y | Z | L |
|---|---|---|---|---|---|
| (93) | 2 | $-CF_3$ | H | H | H |
| (94) | 5 | $-CF_3$ | H | H | H |
| (95) | 4 | $-CF_3$ | H | H | H |
| (96) | 5 | Cl | H | H | H |
| (97) | 2 | Cl | H | H | H |
| (98) | 3 | Cl | H | H | H |
| (99) | 5 | Cl | F | H | H |
| (100) | 2 | Cl | F | H | H |
| (101) | 3 | Cl | F | H | H |
| (102) | 5 | F | H | H | F |
| (103) | 2 | F | H | H | F |
| (104) | 3 | F | H | H | F |
| (105) | 2 | $-CF_3$ | H | H | F |
| (106) | 3 | $-CF_3$ | H | H | F |
| (107) | 5 | $-CF_3$ | H | H | F |
| (108) | 2 | Cl | H | H | F |
| (109) | 3 | Cl | H | H | F |
| (110) | 5 | Cl | H | H | F |

**Patentansprüche**

**1.** Halogenierte Terphenyle der Formel 1

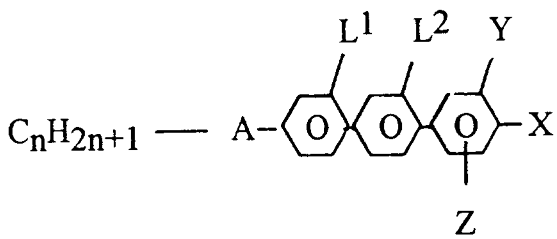

1

worin n 1 bis 7, A trans-1,4-Cyclohexylen, 1,4-Phenylen oder eine Einfachbindung, X Cl oder $-CF_3$ und Y, $L^1$, $L^2$ und Z jeweils unabhängig voneinander H oder F bedeuten wobei mindestens einer der Reste $L^1$, $L^2$ und Y F bedeutet,
der Formel 2

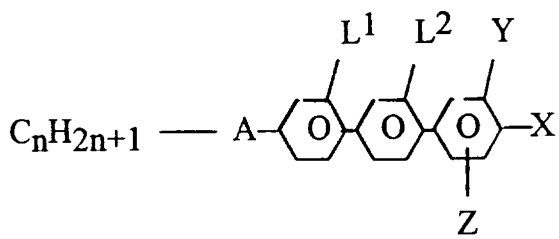

2

worin n 1 bis 7, A trans-1,4-Cyclohexylen oder 1,4-Phenylen, X F, $-OCF_3$ oder $-OCHF_2$ und $Y^1$, $L^1$, $L^2$ und Z jeweils unabhängig voneinander H oder F bedeuten, wobei mindestens einer der Reste $L^1$, $L^2$

und Y F bedeutet,
der Formel 3

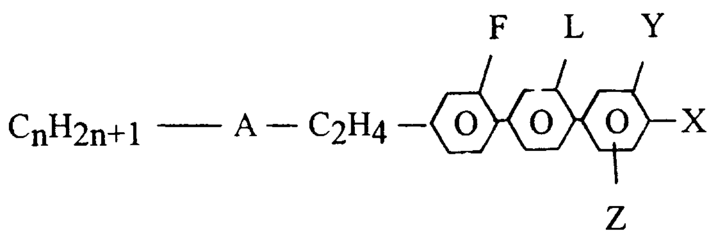

3

worin n 1 bis 7, A trans-1,4-Cyclohexylen oder 1,4-Phenylen, X F, Cl, $-CF_3$, $-OCF_3$ oder $-OCHF_2$ und Y, L und Z jeweils unabhängig voneinander H oder F bedeuten,
der Formel 4

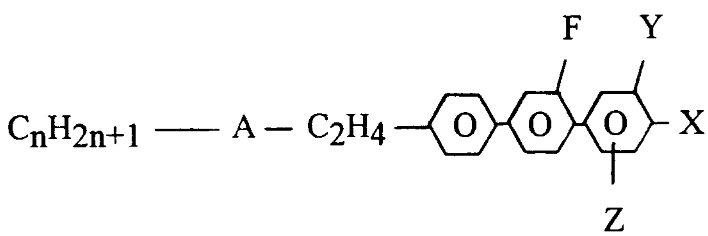

4

worin n 1 bis 7, A trans-1,4-Cyclohexylen oder 1,4-Phenylen, X F, Cl, $-CF_3$, $-OCF_3$ oder $-OCHF_2$ und Y und Z jeweils unabhängig voneinander H oder F bedeuten,
sowie der Formel 5

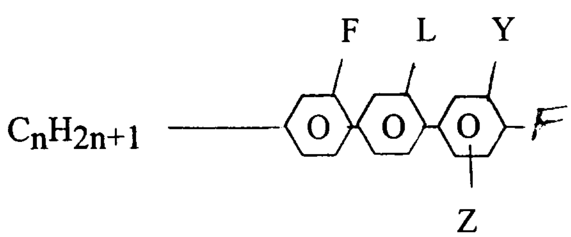

5

worin n 1 bis 7 und Y, L und Z jeweils unabhängig voneinander H oder F bedeuten, mit der Maßgabe, daß L, Y und/oder Z F bedeutet.

**2.** Verwendung der Terphenyle der Formel I nach Anspruch 1 als Komponenten flüssigkristalliner Medien für elektrooptische Anzeigen.

**3.** Flüssigkristallines Medium für elektrooptische Anzeigen mit mindestens zwei flüssigkristallinen Komponenten, dadurch gekennzeichnet, daß mindestens eine Komponente ein Terphenyl der Formel I nach Anspruch 1 ist.

**4.** Elektrooptische Anzeige auf der Basis einer Flüssigkristallzelle, dadurch gekennzeichnet, daß die Flüssigkristallzelle ein Medium nach Anspruch 3 enthält.

## Claims

1. Halogenated terphenyls of the formula 1

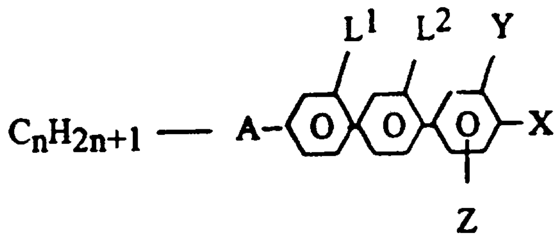

1

in which n is 1 to 7, A is trans-1,4-cyclohexylene, 1,4-phenylene or a single bond, X is Cl or $-CF_3$ and Y, $L^1$, $L^2$ and Z are each, independently of one another, H or F, where at least one of the radicals $L^1$, $L^2$ and Y is F,
of the formula 2

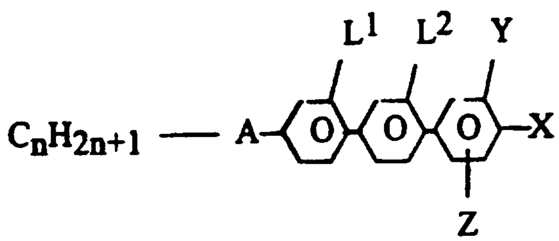

2

in which n is 1 to 7, A is trans-1,4-cyclohexylene or 1,4-phenylene, X is F, $-OCF_3$ or $-OCHF_2$, and $Y^1$, $L^1$, $L^2$ and Z are each, independently of one another, H or F, where at least one of the radicals $L^1$, $L^2$ and Y is F,
of the formula 3

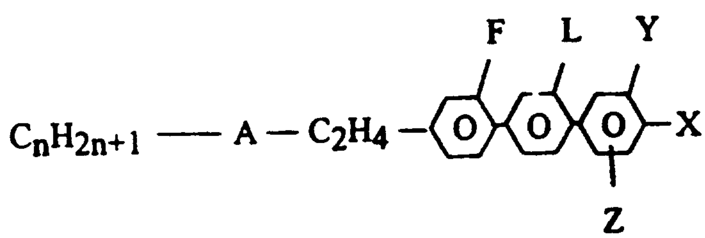

3

in which n is 1 to 7, A is trans-1,4-cyclohexylene or 1,4-phenylene, X is F, Cl, $-CF_3$, $-OCF_3$ or $-OCHF_2$, and Y, L and Z are each, independently of one another, H or F,
of the formula 4

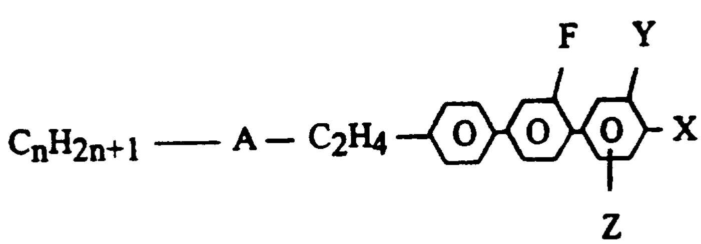

4

in which n is 1 to 7, A is trans-1,4-cyclohexylene or 1,4-phenylene, X is F, Cl, $-CF_3$, $-OCF_3$ or $-OCHF_2$, and Y and Z are each, independently of one another, H or F,
and of the formula 5

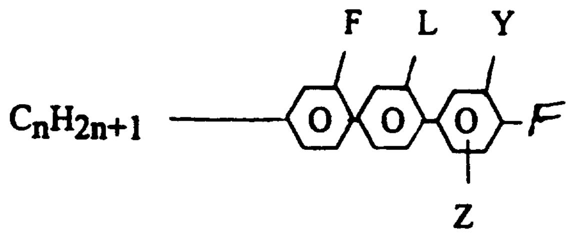

5

in which n is 1 to 7, and Y, L and Z are each, independently of one another, H or F, with the proviso that L, Y and/or Z is F.

**2.** Use of the terphenyls of the formula I according to Claim 1 as components of liquid-crystalline media for electro-optical displays.

**3.** Liquid-crystalline medium for electro-optical displays having at least two liquid-crystalline components, characterized in that at least one component is a terphenyl of the formula I according to Claim 1.

**4.** Electro-optical display based on a liquid-crystal cell, characterized in that the liquid-crystal cell contains a medium according to Claim 3.

**Revendications**

**1.** Terphényles halogénés de formule 1

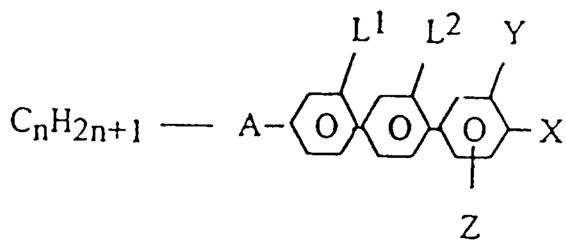

1

où n est compris entre 1 et 7, A représente le trans-1,4-cyclohexylène, le 1,4-phénylène ou une liaison simple, X représente Cl ou $-CF_3$ et Y, $L^1$, $L^2$ et Z représentent, indépendamment les uns des autres, H ou F, au moins l'un des restes $L^1$, $L^2$ et Y représentant F,
de formule 2

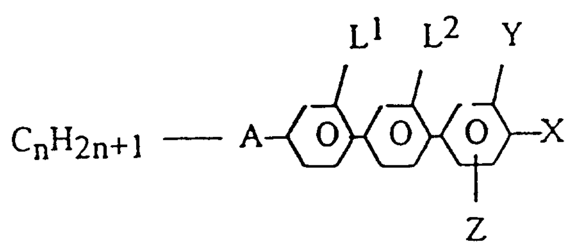

2

où n est compris entre 1 et 7, A représente le trans-1,4-cyclohexylène ou le 1, 4-phénylène, X représente F, $-OCF_3$ ou $-OCHF_2$ et Y', $L^1$, $L^2$ et Z représentent, indépendamment les uns des autres, H ou F, au moins l'un des restes $L^1$, $L^2$ et Y représentant F,

de formule 3

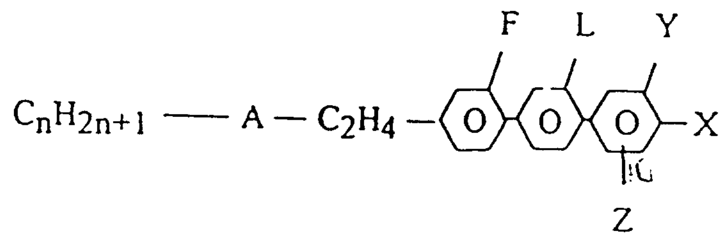

3

où n est compris entre 1 et 7, A représente le trans-1,4-cyclohexylène ou le 1,4-phénylène, X représente F, Cl, $-CF_3$, $-OCF_3$ ou $-OCHF_2$ et Y, L et Z représentent, indépendamment les uns des autres, H ou F,
de formule 4

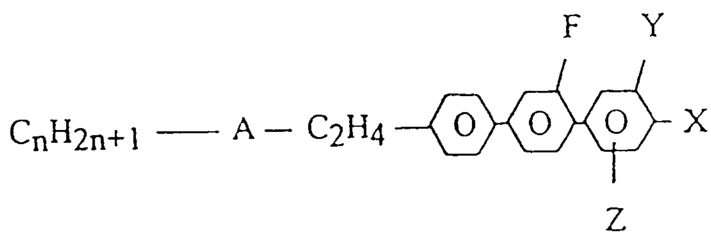

4

où n est compris entre 1 et 7, A représente le trans-1,4cyclohexyléne ou le 1,4-phénylène, X représente F, Cl, $-CF_3$ , $-OCF_3$ ou $-OCHF_2$ et Y et Z représentent, indépendamment l'un de l'autre, H ou F,
ainsi que de formule 5

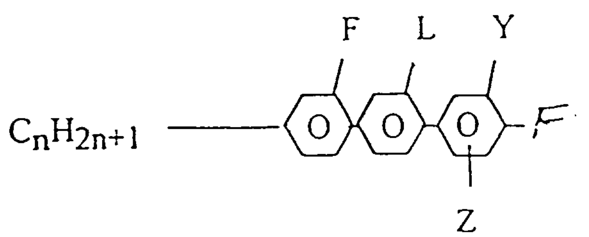

5

où n est compris entre 1 et 7 et Y, L et Z représentent, indépendamment les uns des autres, H ou F, sous réserve que L, Y et/ou Z représentent F.

**2.** Utilisation des terphényles de formule I selon la revendication 1 comme composant de milieux à cristaux liquides pour des dispositifs d'affichage électro-optiques.

**3.** Milieu à cristaux liquides pour des dispositifs d'affichage électro-optiques comportant au moins deux composants à cristaux liquides, caractérisé en ce qu'au moins un composant est un terphényle de formule I selon la revendication 1.

**4.** Dispositif d'affichage électro-optique à base d'une cellule à cristaux liquides, caractérisé en ce que la cellule à cristaux liquides contient un milieu selon la revendication 3.